# EUROPEAN PATENT SPECIFICATION

(11) **EP 2 163 233 B1**
(45) Date of publication and mention of the grant of the patent: **13.07.2011**
(21) Application number: 09011281.4
(22) Date of filing: 02.09.2009
(51) Int. Cl.: A61K 6/06

(54) **Paste-type dental cement**
Pastenähnlicher Dental-Zement
Ciment dentaire pâteux

(30) Priority: 11.09.2008 JP 2008233701
(43) Date of publication of application: 17.03.2010
(73) Proprietor: GC Corporation, Itabashi-ku Tokyo 174-8585 (JP)
(72) Inventor: Kato, Katsuhito, Tokyo 174-8585 (JP); Yarimizu, Hideki, Tokyo 174-8585 (JP)
(74) Representative: Müller-Boré & Partner Patentanwälte

(56) References cited:
- EP-A- 0 988 851
- EP-A- 2 011 468
- US-A1- 2006 247 330
- US-B1- 6 291 548

## Description

The present invention relates to a paste-type dental cement used for temporarily adhering a dental prosthesis or filling a dental cavity to temporarily seal it.

In a dental treatment, a dental prosthesis such as a crown or an inlay is temporarily adhered for several days to several months. At this time, previously used dental cements, which are disclosed in Japanese Patent Application Laid-Open No. 2000-53518, Japanese Patent Application Laid-Open No. 2008-19183, and Japanese Patent Application Laid-Open No. 2008-19246, are used. However, the dental cements are made on the assumption that they are ideally used for permanent restoration by a dental prosthesis which does not fall off from a tooth. Thus, when the permanent dental cements are used for temporarily adhering a dental prosthesis or temporarily filling a cavity, the properties of the dental cements, i.e., adhesiveness and strength of the dental cements become problems when removing the dental prosthesis and the temporary filling.

EP 0 988 851 A2 describes a transparent, elastomeric dental cement composition suitable for temporary or provisional restorations, as well as a method of using the same. The dental cements of EP 0 988 851 includes an oligomer and a prepolymer as polvmerizable materials. In this prior art document, the filler are used to modify the viscosity of the past and eventually to increase the adhesive properties of the cement.

The present invention is directed to provide a paste-type dental cement which is proper for temporary adhering or temporary filling.

The present inventors have carried out earnest works to solve the aforementioned problems. As a result, we have found out that all the aforementioned problems can be solved by a paste-type dental cement comprising a phosphoric acid and/or a polymer of α-β unsaturated carboxylic acid, an oxide powder capable of reacting with the phosphoric acid and/or the polymer of α-β unsaturated carboxylic acid, and a water, wherein the paste-type dental cement includes a liquid not reacting with the oxide powder, and thus completed the present invention

The paste-type dental cement according to the present invention is an excellent cement capable of enabling a dental prosthesis to be easily removed. Further, when this paste-type dental cement is used as a temporary filling agent, the cement does not remain on a tooth surface when after being removed.

A paste-type dental cement according to the present invention basically comprises a phosphoric acid and/or a polymer of α-β unsaturated carboxylic acid, an oxide powder capable of reacting with the phosphoric acid and/or the polymer of α-β unsaturated carboxylic acid, and a water, as an reaction mechanism. Therefore, the paste-type dental cement according to the present invention consists of a first paste in which a liquid component comprising a phosphoric acid and/or a polymer of α-β unsaturated carboxylic acid and a water is blended with a liquid not reacting with the oxide powder, and a second paste in which the oxide powder capable of reacting with the phosphoric acid and/or the polymer of α-β unsaturated carboxylic acid is blended with the water and/or a liquid not reacting with the oxide powder.

The liquid not reacting with the oxide powder is not limited especially. However, water-soluble materials, such as water-soluble organic solvents, e.g., polyhydric alcohol, alcohol, acetone, and dioxane, are preferable when considering a storing property and operativity. Among these, glycerin or polyglycerin such as diglycerin, and polyhydric alcohol such as propylene glycol, dipropylene glycol, sorbitol, mannitol, ethylene glycol, diethylene glycol, or polyethylene glycol, monomethyl ether, are most preferable when considering a safety and operativity.

The polymer of α-β unsaturated carboxylic acid having a weight-average molecular weight of 5,000 to 40, 000 is a polymer of α-β unsaturated monocarboxylic acid or α-β unsaturated dicarboxylic acid. For example, the polymer is a homopolymer or a copolymer of acrylic acid, methacrylic acid, 2-chloroacrylic acid, aconitic acid, mesaconic acid, maleic acid, itaconic acid, fumaric acid, glutaconic acid or citraconic acid. The copolymer could be a copolymer of α-β unsaturated carboxylic acids or a copolymer of α-β unsaturated carboxylic acid and a copolymerizable component with the α-β unsaturated carboxylic acid. In this case, the ratio of α-β unsaturated carboxylic acid is preferably 50% or more. As for the copolymerizable component, for example, acrylamide, acrylonitrile, methacrylic ester, acrylates, vinyl chloride, allyl chloride, and vinyl acetate, can be used. Among these polymers of α-β unsaturated carboxylic acid, a homopolymer or copolymer of acrylic acid or maleic acid is particularly preferable.

The polymer of α-β unsaturated carboxylic acid is a component capable of reacting with the oxide powder and setting. If the polymer having the weight average molecular weight of less than 5,000 is used, strength of the set material is low, and thus there is a problem in durability. Further, adhesiveness to a tooth structure is decreased. If the polymer having the weight average molecular weight of more than 40,000 is used, viscosity of the cement composition is too high, and thus it is very difficult to knead the cement composition. Therefore, the average molecular weight of the polymer of α-β unsaturated carboxylic acid used in the present invention is within the range from 5,000 to 40,000.

As for the oxide powder capable of reacting with the phosphoric acid and/or the polymer of α-β unsaturated carboxylic acid, materials conventionally used for a dental cement can be used. For example, fluoroaluminosilicate powder used for a glass ionomer cement can be used. The fluoroaluminosilicate powder mainly include Al³⁺, Si⁴⁺, F⁻, and O²⁻, and preferably further include Sr²⁺ and/or Ca²⁺. As for the particularly preferable ratio of the main components to the total weight, Al³⁺ is 10 to 21% by weight, Si⁴⁺ is 9 to 21% by weight, F⁻ is 1 to 20% by weight, and the total of Sr²⁺ and Ca²⁺ is 10 to 34% by weight. The surface of the oxide powder capable of reacting with the phosphoric acid and/or the polymer of α-β unsaturated carboxylic acid can be modified with alkoxy silane.

The paste-type dental cement according to the present invention preferably includes a fluorescence agent. When the fluorescence agent is included, a dentist can easily detect a existence of temporarily filled material, can easily remove an excess cement between a dental prosthesis and a gingiva, or can easily remove a temporary cement remaining after removal of a dental prosthesis.

As for the fluorescence agent, an agent making a fluorescent reaction with a light irradiator for dentistry can be used. For example, inorganic fluorescent pigments, e.g., sulfides, silicates, phosphates, tungstates of an alkaline earth metal such as calcium tungstate, magnesium calcium arsenate, barium silicate, calcium phosphate, and calcium zinc phosphate, a phthalic acid derivative (diethyl-2,5-dihydroxyterephthalate, o-phthalaldehyde), a thiophene derivative (2,5-bis(5'-t-butylbenzoxazolyl-2')thiophene, 2,5-bis(6,6'-bis(tert-butyl)-benzooxazol-2-yl)thi ophene), a coumarin derivative (3-phenyl-7-(4-methyl-5-phenyl-1,2,3-triazole-2-y 1)coumarin, 3-phenyl-7-(2H-naphtho[1,2-d]-triazole-2-yl)couma rin), a naphthalimide derivative (N-methyl-5-methoxynaphthalimide), a stilbene derivative (4,4'-bis(diphenyltriazinyl)stilbene, 4,4'-bis(benzoxazol-2-yl)stilbene), and a benzothiazole derivative, can be used. Among these, the phthalic derivative and the thiophene derivative are preferable.

A wavelength peak of the fluorescence agent is preferably 420 nm or less, and more preferably 320 to 400 nm. The content of the fluorescence agent is 0.1 to 3% by weight in a composition after mixing of the paste-type dental cement, and more preferably 0.5 to 2% by weight.

The paste-type dental cement according to the present invention can include powder not reacting with the phosphoric acid and/or the polymer of α-β unsaturated carboxylic acid. As for the cement not reacting, for example, quartz, colloidal silica, feldspar, alumina, strontium glass, barium glass, borosilicate glass, kaolin, talc, calcium carbonate, calcium phosphate, titania, and barium sulfate, can be used. Further, a composite filler acquired by pulverizing a polymer including an inorganic filler can be used. Of course, these can be mixed to be used.

The paste-type dental cement according to the present invention can be properly blended with a coloring agent, a polymerization inhibitor, an ultraviolet absorber, an antibacterial agent, and a perfume.

### [Example]

### <Example>

The present invention will be described in detail below with examples, but the present invention is not limited to these examples.

### [Preparation of fluoroaluminosilicate glass as oxide powder]

Blending amounts of fluoroaluminosilicate glass powders I, II, and III are shown in Table 1.

**[Table 1]**

| | Fluoroaluminosilicate glass powders | | |
|---|---|---|---|
| | I | II | III |
| Aluminium oxide (g) | 21 | 23 | 22 |
| Anhydrous silicic acid (g) | 44 | 91 | 43 |
| Calcium fluoride (g) | 12 | 10 | 12 |
| Calcium phosphate (g) | 14 | 13 | 15 |
| Strontium carbonate (g) | 9 | 13 | 8 |

The fluoroaluminosilicate glass powders I and III were acquired by fully mixing raw materials, holding the mixture in a high temperature electric furnace at 1200°C for 5 hours so as to melt the glass, cooling the mixture after melting the glass, pulverizing the glass using a ball mill for 10 hours, and sieving the pulverized glass powders with 200 meshes (ASTM). The fluoroaluminosilicate glass powder II was acquired by a similar process to that of the fluoroaluminosilicate glass powders I and III except the glass was melted at 1100°C.

### [Preparation of a paste of a dental cement]

Blending amounts of a first paste and a second paste which were used for each example and comparative example are shown in Table 2.

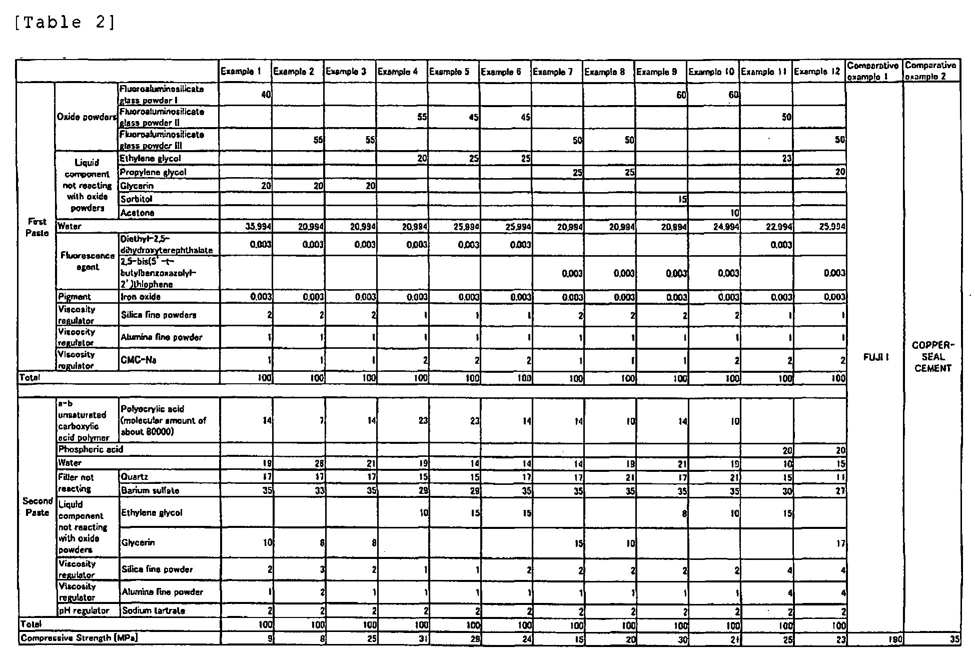

### [Compressive strength]

Since the adhesion strength of a dental cement depends on the strength of the cement itself, the compressive strength was measured to evaluate the retentive strength. The compressive strength was measured according to JIS T6609-1:2005 8.4 (compressive strength). A kneaded dental cement composition was filled in a metal mold having an inner diameter of 4 mm and a length of 6 mm so as to acquire a cylindrical set body as a testing piece. The testing piece was immersed in distilled water at 37°C for 24 hours and subjected to a compressive test at a crosshead speed of 1 mm/min. by a universal testing machine (product name: AUTOGRAPH, produced by SHIMADZU CORPORATION).

### <Examples 1 to 12>

In each example, 0.7g of the first paste and 1.0g of the second paste were weighed and taken on a kneading paper, and kneaded for 30 seconds using a spatula so as to be homogeneously mixed. The compressive strength of the dental cement set body was shown in Table 2.

### <Comparative example 1>

A commercial dental glass ionomer cement (product name: FUJI I, produced by GC Corporation) was used. 1.8g of a cement powder and 1.0g of a cement liquid were weighed and taken on a kneading paper, and kneaded for 1 minute to 1 minute and 30 seconds using a spatula so that the power agent and the liquid agent were homogeneously mixed. The compressive strength was measured by a similar method to that of the examples. The results were shown in the table.

### <Comparative example 2>

A dental temporarily filling cement (product name: COPPER-SEAL CEMENT, produced by GC Corporation) as a commercial zinc phosphate cement was used. 1.5g of a cement powder and 0.5mL of a cement liquid were weighed and taken on a glass kneading plate and kneaded for 1 minute to 1 minute and 30 seconds using a stainless spatula so that the powder agent and the liquid agent were homogeneously mixed. The compressive strength was measured by a similar method to that of the examples. The results were shown in Table 2.

Clearly from Table 2, the dental cements of Examples 1 to 12 have the smaller compressive strength than that of the dental cement of Comparative example 1, and have the equivalent compressive strength to that of COPPER-SEAL CEMENT which was the cement for temporary filling and temporary adhering of Comparative example 2. Therefore, it was confirmed that the dental cements of Examples 1 to 12 were proper for temporary filling or temporary adhering.

## Claims

1. A paste-type dental cement consisting of:
a first paste, comprising:
a fluoroaluminosilicate glass powder;
a liquid component not reacting with the fluoroaluminosilicate glass powder, the liquid being one member selected from the group consisting of polyhydric alcohol, alcohol, acetone, or dioxane;
a water; and
a fluorescence agent; and
a second paste, comprising:
a phosphoric acid and/or a polymer of α-β unsaturated carboxylic acid;
a water; and
a powder not reacting with the phosphoric acid and/or the polymer of α-β unsaturated carboxylic acid.

## Patentansprüche

1. Pastöser Dentalzement, bestehend aus
einer ersten Paste, umfassend
ein Fluoraluminosilikatglaspulver,
einen flüssigen Bestandteil, der nicht mit dem Fluoraluminosilikatglaspulver reagiert, wobei die Flüssigkeit ein Mitglied, ausgewählt aus der Gruppe, bestehend aus mehrwertigem Alkohol, Alkohol, Aceton oder Dioxan, ist,
Wasser und
ein Fluoreszenzmittel, und
einer zweiten Paste, umfassend
eine Phosphorsäure und/oder ein Polymer einer α-β-ungesättigten Carbonsäure,
Wasser, und
ein Pulver, das nicht mit der Phosphorsäure und/oder dem Polymer einer α-β-ungesättigten Carbonsäure reagiert.

## Revendications

1. Un ciment dentaire pâteux consistant en:
une première pâte, comprenant :
une poudre de verre de fluoroaluminosilicate;
un composant liquide ne réagissant pas avec la poudre de verre de fluoroaluminosilicate, le liquide étant un élément sélectionné parmi le groupe consistant en de l'alcool polyhydrique, de l'alcool, de l'acétone ou du dioxan;
une eau; et
un agent de fluorescence; et
une seconde pâte, comprenant:
un acide phosphorique et/ou un polymère d'acide carboxylique insaturé α-β;
une eau; et
une poudre ne réagissant pas avec l'acide phosphorique et/ou le polymère d'acide carboxylique insaturé α-β.
